# EUROPEAN PATENT SPECIFICATION

(11) **EP 3 280 815 B1**
(45) Date of publication and mention of the grant of the patent: **28.10.2020**
(21) Application number: 16717589.2
(22) Date of filing: 11.04.2016
(51) Int. Cl.: C12Q 1/04, G01N 33/573, C07K 16/40, C07K 16/12

(54) **METHOD AND DEVICE FOR DETECTING A CARBAPENEMASE-PRODUCING ENTEROBACTERIACEAE**
VERFAHREN UND VORRICHTUNG ZUM NACHWEIS EINER CARBAPENEMASEPRODUZIERENDEN ENTEROBACTERIACEAE
PROCÉDÉ ET DISPOSITIF DE DÉTECTION D'ENTÉROBACTÉRIES PRODUCTRICES DE CARBAPÉNÉMASE

(30) Priority: 10.04.2015 EP 15163286
(43) Date of publication of application: 14.02.2018
(73) Proprietor: Coris Bioconcept SPRL, 5032 Gembloux (BE)
(72) Inventor: BOGAERTS, Pierre, 5340 Haltinne (BE); GLUPCZYNSKI, Gerald, 1050 Brussels (BE); HUANG, Te-Din, 5000 Namur (BE); MERTENS, Pascal, 5340 Haltinne (BE); OTE, Isabelle, 4000 Liège (BE); LECLIPTEUX, Thierry, 5100 Wépion (BE)
(74) Representative: Pronovem
(86) International application number: PCT/EP2016/057927
(87) International publication number: WO 2016/162564

(56) References cited:
- WO-A1-2011/042454
- WO-A1-2013/011072
- WO-A1-2014/160192
- TOMOE KITAO ET AL: "Development of an immunochromatographic assay for diagnosing the production of IMP-type metallo- -lactamases that mediate carbapenem resistance in", JOURNAL OF MICROBIOLOGICAL METHODS, ELSEVIER, AMSTERDAM, NL, vol. 87, no. 3, 23 September 2011 (2011-09-23), pages 330-337, XP028109164, ISSN: 0167-7012, DOI: 10.1016/J.MIMET.2011.09.011 [retrieved on 2011-10-01]
- HAMMOUDI D ET AL: "How to detect carbapenemase producers? A literature review of phenotypic and molecular methods", JOURNAL OF MICROBIOLOGICAL METHODS, vol. 107, 7 October 2014 (2014-10-07), pages 106-118, XP029098803, ISSN: 0167-7012, DOI: 10.1016/J.MIMET.2014.09.009
- Anonymous: "A0A067XZS9", , 7 January 2015 (2015-01-07), XP055282729, Retrieved from the Internet: URL:http://www.uniprot.org/uniprot/A0A067X ZS9.txt?version=3 [retrieved on 2016-06-22]
- Anonymous: "K4PA55", , 29 October 2014 (2014-10-29), XP055282742, Retrieved from the Internet: URL:http://www.uniprot.org/uniprot/K4PA55. txt?version=8 [retrieved on 2016-06-22]
- Anonymous: "UPI0002ED7F67", , 8 July 2014 (2014-07-08), XP055282750, Retrieved from the Internet: URL:http://www.uniprot.org/uniparc/UPI0002 ED7F67 [retrieved on 2016-06-22]
- Isabelle Ote ET AL: "Development of a novel immunochromatographic confirmatory test for the detection of OXA--48 carbapenemase in Enterobacteriaceae", , 4 May 2015 (2015-05-04), XP055282851, Retrieved from the Internet: URL:http://www.corisbio.com/pdf/Science/OX A-48/Eccmid abstract, 2015.pdf [retrieved on 2016-06-22] -& Anonymous: "No title", , 22 June 2016 (2016-06-22), XP055282858, Retrieved from the Internet: URL:http://citenpl.internal.epo.org/wf/web /citenpl/citenpl.html?_url=http%3A%2F%2Fww w.corisbio.com%2Fpdf%2FScience%2FOXA-48%2F Eccmid%2520abstract%2C%25202015.pdf [retrieved on 2016-06-22]

## Description

### Field of the Invention

The present invention relates to the field of biotechnology and in particular to a method and a device for detecting, and/or identifying, one or several OXA-type Carbapenemase-producing Enterobacteriaceae (CPE), especially selected from the group consisting of OXA-type CPE, KPC-type CPE, VIM-type CPE and NDM-type CPE, more preferably selected from the group consisting of OXA-type CPE and KPC-type CPE, in particular OXA-48 CPE and OXA-48-like CPE.

### Background of the Invention

Carbapenemase-producing Enterobacteriaceae (CPE) constitute a major problem of public health. Indeed, carbapenemase-lactamin antibiotics, in particular carbapenem, are considered to be the last line of effective therapy to treat infections related to multi-resistant gram negative bacteria and because carbapenemases, expressed by such bacteria, are often linked with resistance mechanisms against different other classes of antibiotics.

Usually, the known methods for the detection or identification of Carbapenemase-producing Enterobacteriaceae (CPE) are relatively long to implement, between 48 and 72 hours, including culture steps and phenotypical (comparative growth analysis of the tested bacteria in presence of antibiotics and/or molecular identification assays, and require numerous comparative tests.

Major carbapenemases present in enterobacteria are of the type OXA, KPC, VIM, NDM and IMP, while other carbapenemases, such as SME or IMI, present a lower clinical interest.

Thus, there is a clear clinical need for the development of new methods and means, assays, allowing a rapid and efficient identification, preferably in a delay of less than fifteen minutes, of the major carbapenemases, especially OXA-48 type carbapenemase, that presents a high prevalence in North European countries, and KPC type carbapenemases that that is endemic at least in the United States and Greece, especially methods and means which could be complementary to, or even replace, actual phenotypical and/or molecular detection methods and assays.

### State of the Art

Various methods and assays have been proposed for the detection of carbapenemases as disclosed, for example, in the scientific publication Nordmann et al., Emerg. Infect. Dis., 2012, or in the international patent application WO2013072494, describing an enzymatic method, so called the Carba NP (Carbapenemase Nordmann-Poirel) test, using the acidification of the reactional medium, generated through enzymatic hydrolysis of the imipenem antibiotic, to detect carbapenemases. However, such assay and method require the use of unstable reactive media, careful manipulation to perform the assay, and considerable skill and expertise to avoid false negative or false positive detections.

The international patent application WO 2012/003955 describes a carbapenemase detection method through the use of fluorescent antibiotics that can be hydrolysed by these enzymes. However, if such method is relatively fast, i.e. between 30 minutes and several hours, it requires the use of complex and expensive means, like epifluorescence and cofoncal microscopy, and trained people for performing this assay.

The international patent application WO 2012/143535 describes the detection of carbapenemases by mass spectrometry (MS). However, this method is complex and requires highly qualified people to perform the different method steps and to provide an analysis of the results thus obtained.

Therefore, there is an important need for simple and rapid detection of carbapenemases, especially OXA-48 type and KPC-type carbapenemases, for the detection of resistant enterobacteria that constitute a major public health problem.

### Aims of the Invention

The present invention provides a new method and means for carbapenemases detection and/or identification, especially of OXA, especially OXA-48, KPC, VIM, and/or NDM type carbapenemases, which do not present the drawbacks of the prior art.

The present invention provides method and means that are simple, rapid, and allowing detection and/or identification of carbapenemases, preferably within less than one hour, more preferably within less than thirty minutes and more preferably within less than fifteen minutes.

The present invention provides also means, especially a detection assay and/or a device, which are highly stable at room temperature and could therefore be kept for a long time, e.g. six months, preferably more than one year and, more preferably, more than two years, and used in major clinical microbiological laboratories, without any specific dedicated detection means for detection and results encoding.

A further aim of the present invention is to provide method and means allowing, possibly simultaneously, detection of multiple carbapenemases involved in antibiotic resistance of various bacteria, especially Enterobacteria, preferably with the same detection means, more preferably on the same detection support.

### Summary of the Invention

The invention relates to a method for the detection of one or several carbapenemases from a Carbapenemase-producing Enterobacteriaceae (CPE) possibly present in a biological sample, the method comprising the steps of providing a support, immobilizing on the support one or several capture reagents interacting with one or several first epitope regions of one or several carbapenemases, selected from the group consisting of OXA, especially OXA-48, KPC, VIM, and/or NDM carbapenemases, providing one or several detection reagents interacting with one or several second epitope regions of one or several carbapenemases, selected from the group consisting of OXA, especially OXA-48, KPC, VIM, and/or NDM carbapenemases, the detection reagent(s) being linked, directly or indirectly, to a label to form one or several detection conjugates, providing a sample to be tested comprising, or not, carbapenemase(s) to detect, bringing the sample into contact with the capture reagent(s), revealing a specific binding between carbapenemase(s) present in this sample by the detection reagent(s).

The method according to the invention may further comprise any one, or any suitable combination, of the following features:
- the capture reagent (s) is (are) of the same nature and bind(s) or not to the same carbapenemase or carbapenemases first epitope region or regions as the one(s) (second epitope region(s)) of the detection reagent(s),
- the method further comprises the steps of immobilizing on the support, one or several control reagents to interact with the detection reagent(s), providing one or several control detection reagents to interact with and bind to the control reagent(s), the detection reagent(s) being linked, directly or indirectly, to a label to form one or several control detection conjugates,
- the capture reagent(s), the detection reagent(s) and/or the control reagent (s) are selected from the group consisting of monoclonal antibodies, polyclonal antibodies, portion(s) of antibodies, nanobodies, alphabodies, microantibodies, affilines, affimers, fymomers, affitins or a mixture thereof,
- the label of the detection reagent(s), and/or of the control detection reagent(s), is selected from the group consisting of metallic colloids, latex particles or coloured particles,
- the carbapenemase from a Carbapenemase-producing Enterobacteriaceae (CPE) is an OXA, preferably an OXA-48, type carbapenemase and wherein the capture reagent(s), or possibly the capture reagent(s), is (are) specific and bind(s) to the amino acid sequence or fragments of at least 6, 7, 8, 9, 10, 11, 12, 13, 14, 15, 16 or 17 preferably consecutive amino acids of the following sequence: SEQ.ID.NO3: IRAKTGYSTRIEPKIGWW,
- the method further comprises the step of discriminating between an OXA-163 type carbapenemases and others OXA, especially the OXA-48 type carbapenemases,
- the capture reagent(s) and/or the detection reagent bind(s) the following amino acid sequences or fragments of at least 6, 7, 8, 9, 10, 11 or 12, preferably consecutive amino acids of the following sequences: SEQ.ID.N01: TAMKYSVVPVY or SEQ.ID.N02: SFLRKLYHNKLHV,
- the carbapenemase from a Carbapenemase-producing Enterobacteriaceae (CPE) is a KPC-type carbapenemase and wherein the capture reagent(s) and the detection reagent(s) are specific and bind to the amino acid sequence or fragments of at least 6, 7, 8, 9, 10, 11, 12, 13, 14, 15, 15, 16, 17, 18, 19, 20, 21, 22, 23, 24, 25, 26, 27, 28, 29, 30, 31, 32, or 33, preferably consecutive amino acids, of the following sequence: SEQ.ID.N08: GDKTGTCGVYGTANDYAVVWPTGRAPIVLAVYTR,
- the support is selected from the group consisting of dipsticks or lateral flow devices.

The invention relates also to an immunochromatographic detection device for performing the method steps according to the invention, this device comprising a support comprising one or several capture reagents interacting with one or several first epitope regions of one or several carbapenemases, and comprising one or several detection reagents interacting with one or several second epitope regions of the carbapenemase(s), the detection reagent(s) being linked, directly or indirectly, to a label to form one or several detection conjugates.

The device according to the invention may further comprise in addition to the features mentioned above, any one or any suitable combination, of the following features:
- the device further comprises one or several control reagents to interact with and bind to one or several control detection reagents, and one or several control detection reagents to interact with and bind to the control reagent(s), the detection reagents being linked, directly or indirectly, to a label to form one or several control conjugates,
- the label of the detection reagent(s), and/or the control detection reagent(s), is selected from the group consisting of metallic colloids, latex particles or coloured particles,
- the device is a dipstick or lateral flow device.
- the carbapemenase from a Carbapemenase-producing Enterbactericeae (CPE) is an OXA, preferably an OXA-48 type carbapemenase, and the capture agent(s) is (are) specific and bind(s) to the following amino acid sequence or a fragment of at least 6, 7, 8, 9, 10, 11, 12, 13, 14, 15, 16 or 17 amino acids of the following sequence: SEQ.ID.NO3: IRAKTGYSTRIEPKIGWW,
- the capture reagent(s) and/or the detection reagent(s) bind(s) to one of the following amino acid sequences or a fragment of at least 6, 7, 8, 9, 10 or 11 amino acids of the following sequences: SEQ.ID.N01: TAMKYSVVPVY, SEQ.ID.N02: SFLRKLYHNKLHV,
- the carbapemenase from a Carbapemenase-producing Enterobacteriacae (CPE) is a KPC-type carbapemenase and the capture reagent(s) and the detection reagent(s) are specific and bind(s) to the following amino acid sequence or a fragment of at least 6, 7, 8, 9, 10, 11, 12, 13, 14, 15 16, 17, 18, 19, 20, 21, 22, 23, 24, 25, 26, 27, 28, 29, 30, 31, 32 or 33 amino acids of the following sequence: SEQ.ID.N08: GDKTGTCGVYGTANDYAVVWPTGRAPIVLAVYTR.

The invention relates also to the use of the detection device according to the invention for the detection and/or the identification of OXA, preferably OXA-48 and/or a KPC type carbapenemases from a Carbapenemase-producing Enterobacteriaceae (CPE) in a biological sample.

### Brief Description of the Drawings

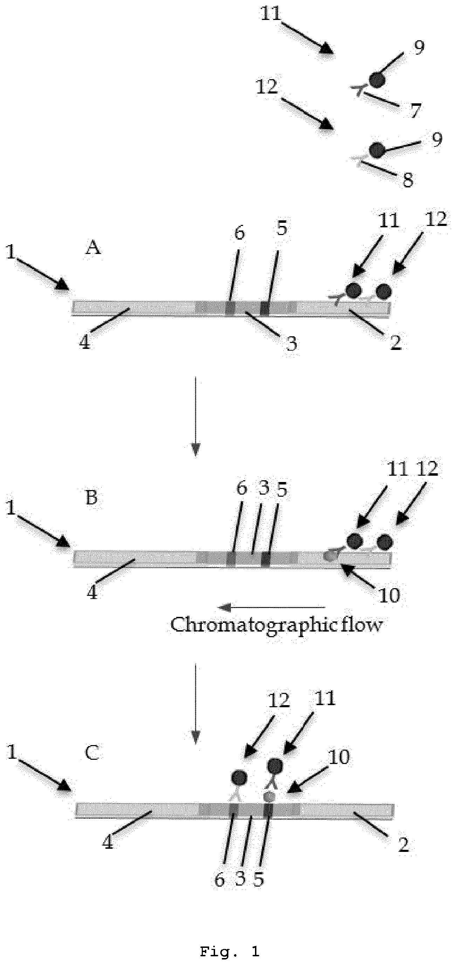

Figure 1 schematically represents the different steps of the method according to the invention wherein the detection is made on a sheet-like immunochromatographic device according to the invention.
Figure 2 schematically represents the detection device according to the invention before the sample has been drop off and the visual results related to positive, negative and invalid tests.

### Detailed description of the invention

The present invention relates to an immunochromatographic method for detecting and/or identifying carbapenemase(s), in particular selected from the group consisting of OXA, KPC, VIM and/or NDM-type type carbapenemase(s), more preferably selected from the group consisting of OXA, especially OXA-48 or OXA-163 and/or KPC type carbapenemase(s), from a Carbapenemase-producing Enterobacteriaceae (CPE) possibly present in a biological sample, such as a clinical sample or a bacterial culture or hemoculture.

Advantageously, the method according to the invention comprises the steps of:
- providing a support 3,
- immobilizing on this support 3, one or several capture reagent(s) 5 interacting with one or several first epitope region(s) of carbapenemase(s) 10 to detect, preferably selected from the group consisting of OXA, especially OXA-48, KPC, VIM, and/or NDM carbapenemase(s),
- providing one or several detection reagents 7 interacting with one or several second epitope regions of the carbapenemase(s), preferably selected from the group consisting of OXA, especially OXA-48, KPC, VIM, and/or NDM carbapenemases, said detection reagent(s) 7 being linked, directly or indirectly, to a label 9 to form one or several detection conjugates 11,
- providing a sample to be tested comprising, or not, one or several carbapenemases 10 to detect,
- bringing this sample into contact with the capture reagent(s) 5,
- revealing a specific binding between the one or several carbapenemases 10 present in the sample by the, preferably labelled, detection reagent(s) 7.

In the present description of the invention, the terms "interact" or "interacting" may be interpreted as being synonyms of "bind" or "binding".

Preferably, one capture reagent 5 interacts with one first epitope region of one carbapenemase. However, one capture reagent 5 may interact with several first epitope regions of one carbapenemase.

Preferably, multiple capture reagents 5, interacting with one or several first epitope region(s) of one specific carbapenemase, may be used to interact with multiple carbapenemases, possibly simultaneously, present in the sample.

According to a preferred embodiment of the invention, the capture reagent(s) interact(s) with the same carbapenemase(s) epitope region(s) as the one(s) of the detection reagent(s).

The method according to the invention may further comprise the steps of:
- immobilizing on the support one or several control reagents 6 interacting with one or several control detection reagents 8,
- providing one or several control detection reagents 8 to interact and bind said one or several control reagents 6, sais control detection reagents 8 being linked, directly or indirectly, to a label 9 to form one or several control detection conjugates 12.

Preferably, the complex(s) formed by the capture reagent(s) 5 and the detection conjugate(s) 11 and/or the one(s) formed by the control reagent(s) 6 and the control detection conjugate(s) 12 generate a detectable signal, preferably a visual or colorimetric signal.

Preferably, the sample is a biological sample comprising a colony from a carbapenemase-producing Enterobacteriaceae (CPE).

Preferably, the step of bringing the sample to analyse into contact with the capture reagent(s) 5, is performed using a buffer solution containing a resuspended bacterial colony, by passive diffusion. If the tested sample contains the carbapenemase 10 to be detected, the complex formed by the carbapenemase and the detection reagent(s) 7 will remain bound to the anti-carbapenemase capture reagent(s) 5 adsorbed onto the support. The result of this specific binding has the advantage of being visible within fifteen minutes in the form of at least a red line that develops on the support. The buffer solution continues to migrate to encounter the control reagent(s) 6 that bind(s) one or several control conjugate(s) 12, thereby producing at least a second red line.

Preferably, the carbapenemase(s) in question is (are) selected from the group consisting of OXA, preferably OXA-48, KPC, VIM, and/or NDM type carbapenemases, more preferably selected from the group consisting of OXA, especially OXA-48, KPC, VIM and NDM type carbapenemases, even more preferably selected from the group consisting of OXA, especially OXA-48, KPC and VIM type carbapenemases, or further more preferably selected from the group consisting of OXA, especially OXA-48 and KPC type carbapenemases.

Preferably, the capture reagent(s) 5 is (are) reagent(s) able to bind and fix specifically or not a first region, an amino acid sequence, of a antigenic structure or structures of one or several carbapenemase(s) to be detected in the sample. Preferably, the capture reagent is a monoclonal or polyclonal antibody, or portion thereof, directed against one or several epitope region(s) of the carbapenemases(s) in question. Preferably, it is any suitable immunoglobulin (Ig).

Preferably, the capture reagent(s) 5 may be a domain antibody(ies) (sdAb), also called nanobody(ies), nanofitine(s), alphabody(ies), microantibody(ies) (i.e. artificial short chain of amino acids copied from a fully functional natural antibody(ies)), affilin(s), affimer(s), affitin(s), fymomer(s), aptamer(s), somamer(s), or any specific capture molecule(s) that can specifically recognize the carbapenemase(s) structure(s) to be detected, or a portion of the structure(s), via suitable complementary regions.

Preferably, the capture reagent(s) 5 is (are) produced by genetic engineering or by synthesis and is (are) screened and selected according to its (their) binding affinity to the carbapenemase(s) structure(s).

The binding of the capture reagent(s) 5 and the support, for example nitrocellulose, can be obtained through a direct or indirect covalent binding.

Preferably, the detection reagent(s) 7 is (are) reagent(s) able to bind and fix specifically a second region, an amino acid sequence, of a antigenic structure or structures of one or several carbapenemase(s) to be detected in the sample. Preferably, it is a (they are) monoclonal or polyclonal antibody(ies), or portion thereof, directed against the second epitope region(s) of the carbapenemases(s) in question. Preferably, it is any suitable immunoglobulin (Ig).

Preferably, the detection reagent(s) 7 is (are) a monoclonal antibody(ies) directed against a second epitope region of the carbapenemase, said second epitope region being different or not from the first epitope region of the carbapenemase bounded by the capture reagent(s) 5.

The detection reagent(s) 7 is (are) linked, directly or indirectly, to a label 9 to form detection conjugate(s) 11.

Preferably, the control reagent(s) 6 is (are) reagent(s) suitable to bind the control detection reagent(s) 8. For example, it could be a monoclonal or polyclonal antibody or antibodies, or a portion thereof. More preferably, it is a goat anti-chicken antibody, or antibodies, more preferably anti-IgY.

Preferably, the control detection reagent(s) 8 is (are) reagent (s) able to bind and fix the control reagent(s) 6, more preferably it is a monoclonal or polyclonal antibody or antibodies, for example chicken antibody or antibodies, preferably IgY.

Preferably, the control detection reagent(s) 8 is (are) linked, directly or indirectly, to a label 9 to form control conjugate(s) 12.

Preferably, the label 9 is selected from the group consisting of metallic colloids, preferably colloidal gold, sulphur, selenium, barium sulphate, iron sulphate, iodate, silver halide, hydrous oxide, metal sulphide, lead selenide, cadmium selenide, metal phosphate, metal ferrite, silica, liposomes or particles, possibly coated with organic or inorganic layers, conjugated or organic latex, cellulose or polystyrene particles and coloured or fluorescent microparticles for an efficient and possibly discriminative colorimetric or fluorimetric detection of one or several (different) carbapenemase(s) or others enzymes involved in antibiotic resistance in gram negative bacteria.

Preferably, the label 9 comprises colloidal gold particles having an average diameter comprised between about 5 nm and about 50 nm, preferably particles having a diameter between about 20 nm and about 40 nm.

Preferably, the label 9 comprises cellulose, polyester and latex particles comprising one or several chemical additional function(s), such as for example a carboxyl, an amino, a hydroxyl and/or a sulphydryl function. In a preferred embodiment, the label 9 comprises carboxylated amino or sulphydryl latex or cellulose particles.

Several cross-linked reagents may also be used for specific coupling of functional groups present on the micro-particles with the amino acid portion of the detection reagent(s) 7 or through the use of other homo- or hetero-bi-functional cross-linking reagents well described in the state of the art.

Preferably, the support has a form of a strip or a stick. It is preferably a sheet-like structure, more preferably a multiple layers structure wherein a primary substance, laminated on a rigid or semi-rigid polymer, for example cellulose, nitrocellulose, cellulose acetate, glass fibres, nylon, acrylic copolymers/nylon, polyethersulfone and polyester. In a preferred embodiment, one portion of the support comprises a membrane made of glass fibres with a conjugate pad made of polyester, another region comprises a membrane made of nitrocellulose and another region comprises a membrane made of cellulose.

The method according to the invention is a ready to use method, fast and easy to implement.

The method according to the invention will be now described, as examples, in respect to OXA type, especially OXA-48 type and KPC-type carbapenemases, but the method steps may also be applied to VIM and/or NDM type carbapenemases using the capture reagent(s) 5 and the detection reagent(s) 7 in relation with the carbapenemases to detect.

The capture reagent(s) 5 and the detection reagent(s) 7 may specifically recognize any suitable epitope region(s) of OXA-48 type carbapenemases, for example OXA-48 from *Klebsiella pneumoniae* as described in SEQ.ID.N04 and SEQ.ID.N05 or any suitable epitope region(s) of KPC type carbapenemases, for example KPC from *Klebsiella pneumoniae* as described in SEQ.ID.N06 and SEQ.ID.N07.

Epitope mapping performed with twelve different mouse monoclonal antibodies (MAT-8377, MAT-8378, MB6, 1B11, 1D12, 1G5, 2A2, 3B11, 3C1, 3G10, 4F9, 4F12) identified these three particular portions of the OXA-48 type carbapenemases amino acid sequence as binding regions.

Thus, in one example, the capture reagent(s) 5 and possibly the detection reagent(s) 7, specifically or not recognize, all or at least a fragment of, these three following portions or epitopes of the OXA type, preferably the OXA-48 or OXA-163 type carbapenemases amino acid sequence or fragments of at least 6, 7, 8, 9, 10, 11, 12, 13, 14, 15, 16 or 17 amino acids, preferably at least 6, 7, 8, 9, 10, 11, 12, 13, 14, 15, 16 or 17 consecutive linear fragments of any of the following sequences:
- SEQ.ID.N01: TAMKYSVVPVY
- SEQ.ID.N02: SFLRKLYHNKLHV
- SEQ.ID.NO3: IRAKTGYSTRIEPKIGWW

The inventors have discovered unexpectedly that the capture reagent 5 directed against the sequence or fragments of the sequence SEQ.ID.N0:3 is specific and can even been used to discriminate between OXA type carbapenemases, especially between OXA-48 type and OXA-163 type carbapennemases. Indeed, MAT-8377 mouse monoclonal antibody can be used as capture reagent 5 and gives detection specificity against the OXA-163 like variants. The OXA-163 amino acid sequence differs (by a deletion of 4 amino acids) from OXA-48 amino acid sequence within the SEQ.ID.N03 region (IRAKTGY**D**T----KIGWW). Advantageously, the MAT-8377 (directed against SEQ.ID.N03) binding specificity allows detecting all OXA-48-like carbapenemases variants and not the OXA-163 like non-carbapenemase variants.

Epitope mapping performed with three different mouse monoclonal antibodies (PF7, NE7 and NA3) identified a particular portion of the KPC type carbapenemases amino acid sequence as binding region.

Thus, in another example, the capture reagent(s) 5 and the detection reagent(s) 7 specifically recognize, all or at least a part of, the following portion or epitope of the KPC type carbapenemases amino acid sequence or fragments of at least 6, 7, 8, 9, 10, 11, 12, 13, 14, 15, 16, 17, 18, 19, 20, 21, 22, 23, 24, 25, 26, 27, 28, 29, 30, 31, 32 or 33 amino acids of the following sequence: SEQ.ID.N08: GDKTGTCGVYGTANDYAVVWPTGRAPIVLAVYTR.

With a purified recombinant OXA-48 protein, the detection limit of the assay based on the method according to the invention has been shown to be 0,125 ng/mL and with a purified recombinant KPC protein, the detection limit of the assay based on the method according to the invention has been shown to be 0,625 ng/mL.

The method of the examples were evaluated in respect to a collection of reference strains.

The OXA-48 *K*-SeT and KPC *K*-SeT were evaluated on a collection of seventy-six fully characterized clinical strains in the National Reference Laboratory for carbapenemase Producing Bacteria (Belgium) (Table 1 and Table 3).

**Table 1:**

| | | | |
|---|---|---|---|
| 76 strains | 28 strains tested positive with the OXA-48 K-SeT | 22 strains carrying the OXA-48 carbapenemase | *Citrobacter braakii, Citrobacter freundii, Enterobacter aerogenes, Enterobacter asburiae, Enterobacter cloacae, Enterobacter kobei, Escherichia coli, Klebsiella oxytoca, Klebsiella pneumoniae, Serratia marcescens* |
| | | | From these 22 strains, 3 *K.* pneumoniae carry another carbapenemase along with OXA-48: KPC, NDM or VIM. |
| | | 6 strains carrying carbapenemase s from the OXA-48 family | OXA-181, OXA-162, OXA-204, OXA-232 (*K. pneumoniae*), OXA-204 and OXA-244 (*E*. *coli*). |
| | 48 strains tested negative with the OXA-48 *K-*SeT | 20 strains carrying a non-OXA-48 carbapenemase | *GES-6, GIM-1, IMP-4, IMP-8, IMP-11,* KPC-2, KPC-3, NDM-1, NDM-5, VIM-1, VIM-4, VIM-27, OXA-23, OXA-24, OXA-25, OXA-26, OXA-58, OXA-198 |
| | | 28 strains not carrying a carbapenemase | Including OXA-163 and OXA-405 |

The OXA-48 *K*-SeT KPC *K*-SeT were validated by comparison with reference molecular methods, including sequencing, in the National Reference Laboratory for Carbapenemase Producing Bacteria (Belgium) in a prospective study performed on 342 non duplicated, suspected CPE consecutive clinical isolates (Table 2 and Table 4).

**Table 2:**

| *Status* | Positive | Negative | Total |
|---|---|---|---|
| *OXA-48 K-SeT* | | | |
| Positive | 129 | 0 | 129 |
| Negative | 0 | 213 | 213 |
| Total | 129 | 213 | 342 |

**Table 3:**

| | | | |
|---|---|---|---|
| 32 strains | 12 strains tested positive with the KPC *K-*SeT | 12 strains carrying the KPC carbapenemase | *Citrobacter koseri, Escherichia coli, Klebsiella oxytoca, Klebsiella pneumoniae* (KPC-2 and KPC-3 positive). |
| | | | From these 12 strains, 1 *K.* pneumoniae carry another carbapenemase along with KPC-3: OXA-48. |
| | 20 strains tested negative with the KPC *K*-SeT | 10 strains carrying a non-KPC carbapenemase | *GIM-1, IMP-4,* NDM-1, VIM-1, VIM-4, OXA-48, OXA-162, OXA-181, OXA-204, OXA-232, OXA-244 |
| | | 10 strains not carrying a carbapenemase | |

**Table 4:**

| *Status* | Positive | Negative | Total |
|---|---|---|---|
| *KPC K-SeT* | | | |
| Positive | 33 | 0 | 33 |
| Negative | 0 | 309 | 309 |
| Total | 33 | 309 | 342 |

To check intra-batch accuracy, i.e. the repeatability of the method according to the invention, the same positive samples and a buffer solution were processed fifteen times on kits of the same production batch in the same experimental conditions. All observed results were confirmed as expected.

To check inter-batch accuracy, i.e. the reproducibility of the method according to the invention, some positive samples and buffer samples were processed on kits from three different production batches. All results were confirmed as expected.

The detection device 1 according to the invention, to implement the method according to the invention, comprises a support, preferably in the form of a strip, comprising capture reagent(s) 5, interacting with one or several first epitope region(s) of one or several carbapenemase(s) 10 in the sample to be analysed, and intended to form a complex with detection reagent(s) 7 linked, directly or indirectly, to a label 9 to form one or several detection conjugate(s) 11, said detection conjugate(s) 11 being detectable as a colorimetric signal.

Preferably, the detection device 1 further comprises one or several control reagent(s) 6, intended to form a complex with one or several control conjugate(s) 12 formed by control detection reagent(s) 8 linked, directly or indirectly, to a label 9.

Preferably, the carbapenemases, the Carbapemenase-producing Enterbactericeae (CPE), the capture reagent(s) 5, the detection reagent(s) 7, the label 9, the control reagent(s) 6 and 8 and the control conjugate(s) 12 are the ones described in respect to the method according to the invention.

Preferably, the detection device 1 according to the invention comprises, as a support, a primary substance laminated on a rigid polymer to form a strip or a stick.

The detection device 1 according to the invention preferably comprises porous polymeric substances that preferably are laminated on a rigid or semi-rigid polymer to provide mechanical strength and make the device 1 easy to handle. The porosity of the polymeric substances can be such that capillary migration of a fluid, e.g. the biological sample, comprising the carbapenemase(s) to be detected, and its components can be achieved, without any hindrance, from the bottom to the top of the detection device 1, moving along with rehydrated conjugates, i.e. detection conjugate(s) 11 and control conjugate(s) 12. This is obtained thanks to hydrophilic properties of the polymers used, such as cellulose, nitrocellulose, cellulose acetate, glass fibres, nylon, acrylic copolymers/nylon, polyethersulfone and polyester.

Preferably, the detection device 1 according to the invention comprises an application region 2, a detection region 3, possibly a control region 6, and optionally an absorbent region 4.

The application region 2 comprises optionally a conjugate pad, for example made of absorbent paper.

The detection region 3 comprises preferably the capture reagent(s) 5 for the detection of particular portion(s) of the carbapenemase(s) in question.

The detection region 3 may further comprise the control reagent(s) 6, preferably a goat anti-chicken antibody, or antibodies, for example an anti-IgY, to specifically bind the control detection reagent(s) 8.

The detection region 3 is preferably made of cellulose, more preferably obtained from Advanced Microdevices Pvt, Ltd or GE Healthcare, Pall and Millipore.

The application region 2 can be made of a rigid polymer to which is adhered a covering absorbent pad, for example made of glass fibres, absorbing and conducting the sample linked to the detection region 3. The glass fibres may further cover a conjugate pad made of polyester that contains the detection conjugate(s) 11 (detection reagent(s) 7 with its label 9) under a dry form. Also this pad could have any suitable characteristics so that the conjugate(s) 11 (detection reagent(s) 7 with its label 9) could be easily rehydrated by the biological sample liquid to allow a complete removal of the rehydrated conjugate(s) 11 and specific reaction of the reagent(s) 7 with the carbapenemase 10 to be detected.

Preferably, the device 1 according to the invention may also comprise an absorbent region 4 that allows aspiration of the sample that has been transported by capillary action to the end of the nitrocellulose layer.

Preferably, absorbent region 4 is made of cellulose and glass fibres, more preferably of cellulose from Advanced Microdevices Pvt, Ldt and of borosilicate glass fibres from GE Healthcare.

In a preferred embodiment, the application region 2 comprises a membrane made of glass fibres with a conjugate pad made of polyester, the detection region 3 comprises a membrane made of nitrocellulose and the absorbent region 4 comprises a membrane made of cellulose.

Preferably, in the detection device 1 according to the invention, the application region 2 and the conjugate pad can be made in the same material.

Preferably, the detection device 1 according to the invention is a dipstick or lateral flow device.

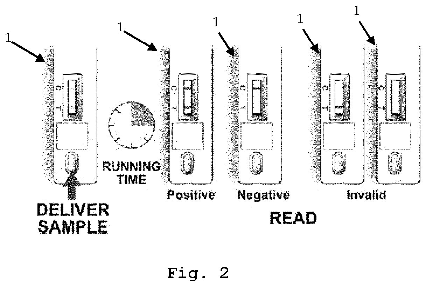

In a preferred embodiment (Figure 2), the detection device 1 comprises a housing comprising the support according to the invention, a first window provided above the application region 2 for the sample deposit, and a second window provided above the detection region 3 to visually read the assay results.

## Claims

1. A method for the detection of one or several OXA carbapenemases from a Carbapenemase-producing Enterobacteriaceae (CPE) possibly present in a biological sample, said method comprising the steps of:
- providing a support (3),
- immobilizing on said support (3) one or several capture reagents (5) interacting with one or several first epitope regions of one or several of the said carbapenemases (10),
- providing one or several detection reagents (7) interacting with one or several second epitope regions of said carbapenemase(s) (10), said detection reagent(s) (7) being linked, directly or indirectly, to a label (9) to form one or several detection conjugates (11),
- providing a sample to be tested comprising, or not, said carbapenemase(s) (10) to detect,
- bringing said sample into contact with the capture reagent or reagents (5),
- revealing a specific binding between the carbapenemase(s) (10) present in said sample by said detection reagent (s) (7) and wherein the capture reagent(s) (5) and possibly the detection reagent(s) (7), is (are) specific and bind(s)
- to the following amino acid sequence or a fragment of at least 6, 7, 8, 9, 10, 11, 12, 13, 14, 15, 16 or 17 amino acids of the sequence: SEQ.ID.N03: IRAKTGYSTRIEPKIGWW.

2. The method according to claim 1, wherein the detection reagent (s) (7) is (are) of the same nature and bind(s) to the same carbapenemase(s) epitope region(s) as the one(s) of the capture reagent(s) (5).

3. The method according to any one of the preceding claims, further comprising the steps of:
- immobilizing on the support one or several control reagents (6) to interact with one or several control detection reagents (8),
- providing one or several control detection reagents (8) to interact with and bind to said control reagent (s) (6), said control detection reagent(s) (8) being linked, directly or indirectly, to a label (9) to form one or several control detection conjugates (12).

4. The method according to any one of the preceding claims, wherein the capture reagent(s) (5), the detection reagent(s) (7) and/or the control reagent(s) (6) are selected from the group consisting of monoclonal antibodies, polyclonal antibodies, portion(s) of antibodies, nanobodies, alphabodies, microantibodies, affilines, affimers, fymomers, affitins or a mixture thereof.

5. The method according to any one of the preceding claims, wherein the control detection reagent (6) is (are) monoclonal or polyclonal antibody or antibodies.

6. The method according to any one of the preceding claims, wherein the label (9) of the detection reagent(s) (7) and/or the control detection reagent(s) (6) is selected from the group consisting of metallic colloids, latex particles or coloured particles.

7. The method of claim 1, further comprising the step of discriminating between OXA-163-type carbapenemases and OXA-48-type carbapenemases.

8. The method according to any one of the preceding claims, wherein the support is selected from the group consisting of dipsticks or lateral flow devices.

9. An immunochromatographic detection device (1) comprising a support (3) comprising one or several capture reagents (5) interacting with one or several first epitope regions of one or several carbapenemases, one or several detection reagents (7) interacting with one or several second epitope regions of said carbapenemase(s), said detection reagent(s) (7) being linked, directly or indirectly, to a label (9) to form one or several detection conjugates (11) and wherein the capture reagent(s) (5), and possibly the detection reagent (s) (7) is (are) specific and bind(s) to the following amino acid sequence or a fragment of at least 6, 7, 8, 9, 10, 11, 12, 13, 14, 15, 16 or 17 amino acids of the sequence: SEQ.ID.NO3: IRAKTGYSTRIEPKIGWW.

10. The immunochromatographic detection device (1) according to the claim 9, further comprising one or several control reagents (6) to interact with and bind to one or several control detection reagents (8), said detection reagent(s) (8) being linked, directly or indirectly, to a label (9) to form one or several control conjugates (12).

11. The immunochromatographic detection device (1) according to the claim 9 or the claim 10, wherein the label (9) of the detection reagent(s) (7) and/or the control detection reagent(s) (8) is selected from the group consisting of metallic colloids, latex particles or coloured particles.

12. The immunochromatographic detection device (1) according to any one of the preceding claims 9 to 11, being a dipstick or lateral flow device.

13. The use of the immunochromatographic detection device (1) according to any one of the preceding claims 9 to 12 for the detection and/or the identification of an OXA carbapenemase from a Carbapenemase-producing Enterobacteriaceae (CPE) in a biological sample.

## Patentansprüche

1. Verfahren zum Nachweis einer oder mehrerer OXA-Carbapenemasen aus einem Carbapenemase erzeugenden Mitglied der Enterobacteriaceae (CPE), das möglicherweise in einer biologischen Probe vorliegt, wobei das Verfahren die Schritte umfasst:
- Vorsehen eines Trägers (3);
- Immobilisieren mindestens eines Fängerreagenz (5) das mit einem oder mehreren ersten Epitopregionen einer oder mehrerer der Carbapenemasen (10) in Wechselwirkung tritt, auf dem Träger (3),
Vorsehen mindestens eines Nachweisreagenz (7), das mit einem oder mehereren Epitopregionen der Carbapenemase(n) (10) in Wechselwirkung tritt, wobei das/die Nachweisreagenz(ien) (7) mittelbar oder unmittelbar mit einer Markierung (9) verbunden ist/sind, um mindestens ein Nachweiskonjugat (11) zu bilden,
- Vorsehen einer zu untersuchenden Probe, die die nachzuweisende(n) Carbapenemase(n) (10) möglicherweise umfasst,
- Kontaktieren der Probe mit dem mindestens einen Fängerreagenz (5),
- Offenbaren einer spezifischen Bindung zwischen der in der Probe vorhandenen mindestens einen Carbapenemase (10) durch das mindestens eine Nachweisreagenz (7), wobei das mindestens eine Fängerreagenz (5) spezifisch ist und sich an folgende Aminosäusresequenz oder ein mindestens 6, 7, 8, 9, 10, 11, 12, 13, 14, 15, 16 oder 17 Aminosäuren umfassendes Fragment der Sequenz bindet: SEQ.ID.NR:3: IRAKTGYSTRIEPKIGWW.

2. Verfahren nach Anspruch 1, wobei das mindestens eine Fängerreagenz (5) der gleichen Art ist und sich an dieselbe Epitopregion von Carbapenemasen bindet wie das mindestens eine Nachweisreagenz (7).

3. Verfahren nach einem der vorstehenden Ansprüche, ferner umfassend die Schritte:
- Immobilisieren mindestens eines Kontrollreagenz (6) auf dem Träger, damit dieses mit mindestens einem Kontroll-Nachweisreagenz (8) in Wechselwirkung tritt,
- Vorsehen mindestens eines Kontroll-Nachweisreagenz (8), damit dieses mit dem Kontrollreagenz (6) in Wechselwirkung tritt und sich daran bindet,
wobei das Kontroll-Nachweisreagenz (8) mittelbar oder unmittelbar mit einer Markierung (9) verbunden ist, um mindestens ein Kontroll-Nachweiskonjugat (12) zu bilden.

4. Verfahren nach einem der vorstehenden Ansprüche, wobei das mindestens eine Fängerreagenz (5), das mindestens eine Nachweisreagenz (7) und/oder das mindestens eine Kontrollreagenz (6) aus folgender Gruppe gewählt sind: monoklonale Antikörper, polyklonale Antikörper, Anteile von Antikörpern, Nanobodies, Alphabodies, Mikroantikörper, Affiline, Affimere, Affimere, Fymomere, Affitine oder eine Mischung davon.

5. Verfahren nach einem der vorehenden Ansprüche, wobei das midestens eine Kontoll-Nachweisreagenz aus folgender Gruppe Gewählt sind Monoklonale Antikörper oder polyklonale Antiköper.

6. Verfahren nach einem der vorstehenden Ansprüche, wobei die Markierung (9) des mindestens einen Nachweisreagenz (7) und/oder des mindestens einen Kontroll-Nachweisreagenz (6) aus folgender Gruppe gewählt ist: metallische Kolloide, Latexpartikel oder gefärbte Partikel.

7. Verfahren nach Anspruch 1, ferner umfassend den Schritt: Unterscheiden zwischen Carbapenemasen vom Typ OXA-163 und Carbapenemasen vom Typ OXA-48.

8. Verfahren nach einem der vorstehenden Ansprüche, wobei der Träger aus der Gruppe der Messstäbe und LFDs (Lateral Flow Devices) gewählt ist.

9. Immunchromatographische Nachweisvorrichtung (1), umfassend: einen Träger (3), umfassend mindestens ein Fängerreagenz (5), das mit mindestens einer ersten Epitopregion mindestens einer Carbapenemase in Wechselwirkung tritt, mindestens ein Nachweisreagenz (7), das mit einem oder mehereren Epitopregionen der Carbapenemase(n) (10) in Wechselwirkung tritt, wobei das/die Nachweisreagenz(ien) (7) mittelbar oder unmittelbar mit einer Markierung (9) verbunden ist/sind, um mindestens ein Nachweiskonjugat (11) zu bilden, und wobei das mindestens eine Fängerreagenz (5) spezifisch ist und sich an folgende Aminosäuresequenz oder ein mindestens 6, 7, 8, 9, 10, 11, 12, 13, 14, 15, 16 17 Aminosäuren umfassendes Fragment der Sequenz bindet: SEQ.ID.NR:3: IRAKTGYSTRIEPKIGWW.

10. Immunchromatographische Nachweisvorrichtung (1) nach Anspruch 9, ferner umfassend: oder mindestens ein Kontrollreagenz (6), das mit mindestens einem Kontroll-Nachweisreagenz (8) in Wechselwirkung treten und sich daran binden soll, mindestens ein Kontroll-Nachweisreagenz (8), das mit dem Kontrollreagenz (6) in Wechselwirkung treten und sich daran binden soll, wobei das mindestens eine Nachweisreagenz (8) mittelbar oder unmittelbar mit einer Markierung (9) verbunden ist, um mindestens ein Kontrollkonjugat (12) zu bilden.

11. Immunchromatographsiche Nachweisvorrichtung (1) nach Anspruch 9 oder - 10, wobei die Markierung (9) des mindestens einen Nachweisreagenz (7) und/oder des mindestens einen Kontroll-Nachweisreagenz (8) aus folgender Gruppe gewählt ist: metallische Kolloide, Latexpartikel oder gefärbte Partikel.

12. Immunchromatographische Nachweisvorrichtung (1) nach einem der Ansprüche 9 - 11, wobei es sich um einen Messstab oder eine LFD handelt.

13. Verwendung der immunchromatographischen Nachweisvorrichtung (1) nach einem der Ansprüche 9 - 12 zu Nachweis und/oder Erkennung einer OXA-Carbapenemase aus einem Carbapenemase erzeugenden Mitglied der Enterobacteriaceae in einer biologischen Probe.

## Revendications

1. Procédé de détection d'une ou plusieurs carbapénémases de type OXA à partir d'entérobactéries productrices de carbapénémase (EPC) éventuellement présentes dans un échantillon biologique, ledit procédé comprenant les étapes consistant à:
- fournir un support (3),
- immobiliser sur ledit support (3) un ou plusieurs réactifs de capture (5) interagissant avec un ou plusieurs épitopes primaires d'une ou plusieurs des dites carbapénémases (10),
- fournir un ou plusieurs réactifs de détection (7) interagissant avec un ou plusieurs épitopes secondaires des dites carbapénémase (s) (10), le ou les réactifs de détection (7) étant lié(s), directement ou indirectement, à un marqueur (9) pour former un ou plusieurs conjugués de détection (11),
- fournir un échantillon à tester comprenant, ou non, la ou les carbapénémases (10) à détecter,
- mettre ledit échantillon en contact avec le ou les réactifs de capture (5),
- révéler une liaison spécifique entre la ou les carbapénémases (10) présentes dans ledit échantillon par ledit ou lesdits réactifs de détection (7) et dans laquelle le ou les réactifs de capture (5) et éventuellement le ou les réactif(s) de détection est (sont) spécifique (s) et se lie (nt) à la séquence d'acides aminés suivante ou à un fragment d'au moins 6,7,8,9,10,11,12,13,14, 15, 16 ou 17 acides aminés de la séquence: SEQ.ID.NO3: IRAKTGYSTRIEPKIGWW.

2. Procédé selon la revendication 1, dans lequel le ou les réactifs de détection (7) sont de même nature et se lie (nt) aux mêmes épitopes de(s) carbapénémase(s) que celle(s) du ou des réactifs de capture (5).

3. Procédé selon l'une des quelconques revendications précédentes, comprenant en outre les étapes consistant à:
- immobiliser sur le support un ou plusieurs réactifs contrôles (6) pour interagir avec un ou plusieurs réactifs contrôles de détection (8),
- fournir un ou plusieurs réactifs contrôles de détection (8) pour interagir et se lier avec le(s)dit(s) réactif(s) contrôles (6), le(s)dit(s) réactifs contrôles de détection (8) étant liés, directement ou indirectement, à un marqueur (9) pour former un ou plusieurs conjugués contrôles de détection (12) .

4. Procédé selon l'une des quelconques revendications précédentes, dans lequel le ou les réactifs de capture (5), le ou les réactifs de détection (7) et/ou le ou les réactifs contrôles (6) sont choisis parmi le groupe constitué par des anticorps monoclonaux, des anticorps polyclonaux, des portions d'anticorps, des nanobodies, des alphabodies, des micro-anticorps, des affilines, des affimères, des fymomères, des affitines ou un mélange de ceux-ci.

5. Procédé selon l'une quelconque des revendications précédentes, dans lequel le ou les réactifs contrôles de détection (8) sont choisis parmi le groupe constitué par des anticorps monoclonaux ou des anticorps polyclonaux.

6. Procédé selon l'une des quelconques revendications précédentes, dans lequel le marqueur (9) du ou des réactif (s) de détection (7) et/ou du (des) réactif (s) contrôles de détection (6) est sélectionné dans le groupe constitué des colloïdes métalliques, des particules de latex ou des particules colorées.

7. Procédé selon la revendication 1, comprenant en outre l'étape de discrimination entre les carbapénémases de type OXA-163 et les carbapénémases de type OXA-48.

8. Procédé selon l'une des quelconques revendications précédentes, dans lequel le support est choisi dans le groupe des dispositifs constitué par des tigettes réactionnelles individuelles de type dipstick ou des tigettes réactionnelles avec migration latérale du flux.

9. Dispositif de détection immunochromatographique (1) comprenant un support (3) comprenant un ou plusieurs réactifs de capture (5) interagissant avec un ou plusieurs épitopes primaires d'une ou plusieurs carbapénémases, un ou plusieurs réactifs de détection (7) interagissant avec un ou plusieurs épitopes secondaires de(s) la dite(s) carbapénémase (s), le(s)dit (s) réactif (s) de détection (7)) étant liés, directement ou indirectement, à un marqueur (9) pour former un ou plusieurs conjugués de détection (11) et dans lequel le ou les réactifs de capture (5) ert éventuellement le ou les réactif(s) de détection est (sont) spécifique (s) et se lie (nt) à
- la séquence d'acides aminés suivante ou à un fragment d'au moins 6, 7, 8, 9, 10, 11, 12, 13, 14, 15, 16 ou 17 acides aminés de la séquence: SEQ.ID.NO3: IRAKTGYSTRIEPKIGWW.

10. Dispositif de détection immunochromatographique (1) selon la revendication 9, comprenant en outre un ou plusieurs réactifs contrôles (6) pour interagir avec et se lier à un ou plusieurs réactifs contrôles de détection (8), ledit ou lesdits réactifs contrôles de détection (8) étant liés, directement ou indirectement à un marqueur (9) pour former un ou plusieurs conjugués contrôles de détection (12).

11. Dispositif de détection immunochromatographique (1) selon la revendication 9 ou la revendication 10, dans lequel le marqueur (9) du ou des réactifs de détection (7) et/ou du ou des réactifs contrôles de détection (8) est choisi dans le groupe constitué par des colloïdes métalliques, des particules de latex ou des particules colorées.

12. Dispositif de détection immunochromatographique (1) selon l'une des quelconques précédentes revendications 9 à 11, qui est une tigette réactionnelle individuelle de type dipstick ou une tigette réactionnelle avec migration latérale du flux.

13. Utilisation d'un dispositif de détection immunochromatographique (1) selon l'une des quelconques précédentes revendications précédentes 9 à 12 pour la détection et/ou l'identification d'une carbapénémase de type OXA à partir d'entérobactéries productrices de carbapénémases (EPC) dans un échantillon biologique.


### SEQUENCE LISTING

<110> Coris BioConcept
<120> METHOD AND DEVICE FOR DETECTING A CARBAPENEMASE PRODUCING ENTEROBACTERIACEAE
<130> BPCORI0005PC
<160> 8
<170> PatentIn version 3.5
<210> 1
   <211> 11
   <212> PRT
   <213> Enterobacteriaceae
<400> 1
   Thr Ala Met Lys Tyr Ser Val Val Pro Val Tyr
   1               5                   10
<210> 2
   <211> 13
   <212> PRT
   <213> Enterobacteriaceae
<400> 2
   Ser Phe Leu Arg Lys Leu Tyr His Asn Lys Leu His Val
   1               5                   10
<210> 3
   <211> 18
   <212> PRT
   <213> Enterobacteriaceae
<400> 3
   Ile Arg Ala Lys Thr Gly Tyr Ser Thr Arg Ile Glu Pro Lys Ile Gly
   1               5                   10                  15
   Trp Trp
<210> 4
   <211> 798
   <212> DNA
   <213> Klebsiella pneumoniae
<400> 4
   atgcgtgtat tagccttatc ggctgtgttt ttggtggcat cgattatcgg aatgcctgcg      60
   gtagcaaagg aatggcaaga aaacaaaagt tggaatgctc actttactga acataaatca     120
   cagggcgtag ttgtgctctg gaatgagaat aagcagcaag gatttaccaa taatcttaaa     180
   cgggcgaacc aagcattttt acccgcatct acctttaaaa ttcccaatag cttgatcgcc     240
   ctcgatttgg gcgtggttaa ggatgaacac caagtcttta agtgggatgg acagacgcgc     300
   gatatcgcca cttggaatcg cgatcataat ctaatcaccg cgatgaaata ttcagttgtg     360
   cctgtttatc aagaatttgc ccgccaaatt ggcgaggcac gtatgagcaa gatgctacat     420
   gctttcgatt atggtaatga ggacatttcg ggcaatgtag acagtttctg gctcgacggt     480
   ggtattcgaa tttcggccac ggagcaaatc agctttttaa gaaagctgta tcacaataag     540
   ttacacgtat cggagcgcag ccagcgtatt gtcaaacaag ccatgctgac cgaagccaat     600
   ggtgactata ttattcgggc taaaactgga tactcgacta gaatcgaacc taagattggc     660
   tggtgggtcg gttgggttga acttgatgat aatgtgtggt tttttgcgat gaatatggat     720
   atgcccacat cggatggttt agggctgcgc caagccatca caaaagaagt gctcaaacag     780
   gaaaaaatta ttccctag                                                   798
<210> 5
   <211> 265
   <212> PRT
   <213> Klebsiella pneumoniae
<400> 5
   Met Arg Val Leu Ala Leu Ser Ala Val Phe Leu Val Ala Ser Ile Ile
   1               5                   10                  15
   Gly Met Pro Ala Val Ala Lys Glu Trp Gln Glu Asn Lys Ser Trp Asn
               20                  25                  30
   Ala His Phe Thr Glu His Lys Ser Gln Gly Val Val Val Leu Trp Asn
           35                  40                  45
   Glu Asn Lys Gln Gln Gly Phe Thr Asn Asn Leu Lys Arg Ala Asn Gln
       50                  55                  60
   Ala Phe Leu Pro Ala Ser Thr Phe Lys Ile Pro Asn Ser Leu Ile Ala
   65                  70                  75                  80
   Leu Asp Leu Gly Val Val Lys Asp Glu His Gln Val Phe Lys Trp Asp
                   85                  90                  95
   Gly Gln Thr Arg Asp Ile Ala Thr Trp Asn Arg Asp His Asn Leu Ile
               100                 105                 110
   Thr Ala Met Lys Tyr Ser Val Val Pro Val Tyr Gln Glu Phe Ala Arg
           115                 120                 125
   Gln Ile Gly Glu Ala Arg Met Ser Lys Met Leu His Ala Phe Asp Tyr
       130                 135                 140
   Gly Asn Glu Asp Ile Ser Gly Asn Val Asp Ser Phe Trp Leu Asp Gly
   145                 150                 155                 160
   Gly Ile Arg Ile Ser Ala Thr Glu Gln Ile Ser Phe Leu Arg Lys Leu
                   165                 170                 175
   Tyr His Asn Lys Leu His Val Ser Glu Arg Ser Gln Arg Ile Val Lys
               180                 185                 190
   Gln Ala Met Leu Thr Glu Ala Asn Gly Asp Tyr Ile Ile Arg Ala Lys
           195                 200                 205
   Thr Gly Tyr Ser Thr Arg Ile Glu Pro Lys Ile Gly Trp Trp Val Gly
       210                 215                 220
   Trp Val Glu Leu Asp Asp Asn Val Trp Phe Phe Ala Met Asn Met Asp
   225                 230                 235                 240
   Met Pro Thr Ser Asp Gly Leu Gly Leu Arg Gln Ala Ile Thr Lys Glu
                   245                 250                 255
   Val Leu Lys Gln Glu Lys Ile Ile Pro
               260                 265
<210> 6
   <211> 960
   <212> DNA
   <213> Klebsiella pneumoniae
<400> 6
   atgtcactgt atcgccgtct agttctgctg tcttgtctct catggccgct ggctggcttt      60
   tctgccaccg cgctgaccaa cctcgtcgcg gaaccattcg ctaaactcga acaggacttt     120
   ggcggctcca tcggtgtgta cgcgatggat accggctcag gcgcaactgt aagttaccgc     180
   gctgaggagc gcttcccact gtgcagctca ttcaagggct ttcttgctgc cgctgtgctg     240
   gctcgcagcc agcagcaggc cggcttgctg gacacaccca tccgttacgg caaaaatgcg     300
   ctggttccgt ggtcacccat ctcggaaaaa tatctgacaa caggcatgac ggtggcggag     360
   ctgtccgcgg ccgccgtgca atacagtgat aacgccgccg ccaatttgtt gctgaaggag     420
   ttgggcggcc cggccgggct gacggccttc atgcgctcta tcggcgatac cacgttccgt     480
   ctggaccgct gggagctgga gctgaactcc gccatcccag gcgatgcgcg cgatacctca     540
   tcgccgcgcg ccgtgacgga aagcttacaa aaactgacac tgggctctgc actggctgcg     600
   ccgcagcggc agcagtttgt tgattggcta aagggaaaca cgaccggcaa ccaccgcatc     660
   cgcgcggcgg tgccggcaga ctgggcagtc ggagacaaaa ccggaacctg cggagtgtat     720
   ggcacggcaa atgactatgc cgtcgtctgg cccactgggc gcgcacctat tgtgttggcc     780
   gtctacaccc gggcgcctaa caaggatgac aagtacagcg aggccgtcat cgccgctgcg     840
   gctagactcg cgctcgaggg attgggcgtc aacgggcagt aaggctctga aaatcatcta     900
   ttggcccacc accgccgccc ttgcgggcgg catggattac caaccactgt cacatttagg     960
<210> 7
   <211> 293
   <212> PRT
   <213> Unknown
<220>
   <223> Complete sequence of the protein KPC-3
<400> 7
   Met Ser Leu Tyr Arg Arg Leu Val Leu Leu Ser Cys Leu Ser Trp Pro
   1               5                   10                  15
   Leu Ala Gly Phe Ser Ala Thr Ala Leu Thr Asn Leu Val Ala Glu Pro
               20                  25                  30
   Phe Ala Lys Leu Glu Gln Asp Phe Gly Gly Ser Ile Gly Val Tyr Ala
           35                  40                  45
   Met Asp Thr Gly Ser Gly Ala Thr Val Ser Tyr Arg Ala Glu Glu Arg
       50                  55                  60
   Phe Pro Leu Cys Ser Ser Phe Lys Gly Phe Leu Ala Ala Ala Val Leu
   65                  70                  75                  80
   Ala Arg Ser Gln Gln Gln Ala Gly Leu Leu Asp Thr Pro Ile Arg Tyr
                   85                  90                  95
   Gly Lys Asn Ala Leu Val Pro Trp Ser Pro Ile Ser Glu Lys Tyr Leu
               100                 105                 110
   Thr Thr Gly Met Thr Val Ala Glu Leu Ser Ala Ala Ala Val Gln Tyr
           115                 120                 125
   Ser Asp Asn Ala Ala Ala Asn Leu Leu Leu Lys Glu Leu Gly Gly Pro
       130                 135                 140
   Ala Gly Leu Thr Ala Phe Met Arg Ser Ile Gly Asp Thr Thr Phe Arg
   145                 150                 155                 160
   Leu Asp Arg Trp Glu Leu Glu Leu Asn Ser Ala Ile Pro Gly Asp Ala
                   165                 170                 175
   Arg Asp Thr Ser Ser Pro Arg Ala Val Thr Glu Ser Leu Gln Lys Leu
               180                 185                 190
   Thr Leu Gly Ser Ala Leu Ala Ala Pro Gln Arg Gln Gln Phe Val Asp
           195                 200                 205
   Trp Leu Lys Gly Asn Thr Thr Gly Asn His Arg Ile Arg Ala Ala Val
       210                 215                 220
   Pro Ala Asp Trp Ala Val Gly Asp Lys Thr Gly Thr Cys Gly Val Tyr
   225                 230                 235                 240
   Gly Thr Ala Asn Asp Tyr Ala Val Val Trp Pro Thr Gly Arg Ala Pro
                   245                 250                 255
   Ile Val Leu Ala Val Tyr Thr Arg Ala Pro Asn Lys Asp Asp Lys Tyr
               260                 265                 270
   Ser Glu Ala Val Ile Ala Ala Ala Ala Arg Leu Ala Leu Glu Gly Leu
           275                 280                 285
   Gly Val Asn Gly Gln
       290
<210> 8
   <211> 34
   <212> PRT
   <213> Unknown
<220>
   <223> Epitope region (231-264)
<400> 8
   Gly Asp Lys Thr Gly Thr Cys Gly Val Tyr Gly Thr Ala Asn Asp Tyr
   1               5                   10                  15
   Ala Val Val Trp Pro Thr Gly Arg Ala Pro Ile Val Leu Ala Val Tyr
               20                  25                  30
   Thr Arg